# EUROPEAN PATENT APPLICATION

(11) **EP 4 306 501 A1**
(43) Date of publication of application: **17.01.2024**
(21) Application number: 22767266.4
(22) Date of filing: 11.03.2022
(51) Int. Cl.: C07B 51/00, C07H 5/06, C07C 29/00, C07D 209/04, C07C 33/22, C07D 213/30, C07D 233/54, C07D 275/02, C07D 309/06, A61K 47/54, C07D 333/16, C07D 239/26, C07D 473/00, C07D 307/42, C07H 3/02, C08B 37/00, C07H 3/06

(54) **NOVEL DEALKOXYPHENYLATION REACTION**

(30) Priority: 12.03.2021 JP 2021040647
(71) Applicant: Daiichi Sankyo Company, Limited, Tokyo 103-8426 (JP)
(72) Inventor: UEDA, Tsuyoshi, Tokyo 103-8426 (JP); NAKAMURA, Tatsuya, Tokyo 103-8426 (JP)
(74) Representative: Marks & Clerk LLP
(86) International application number: PCT/JP2022/010839
(87) International publication number: WO 2022/191312

(57) **Abstract**

To provide a method for obtaining a dealkoxyphenylation product with a high yield from a substrate such as a sugar bound to an alkoxyphenyl group through an oxygen atom. A dealkoxyphenylation product can be obtained with a high yield under mild conditions by reacting a substrate that is bound to a phenyl group substituted by C₁ to C₅ alkoxy at the para- or ortho-position through an oxygen atom with λ3-iodane in a fluorous alcohol and water.

## Description

### Technical Field

The present invention relates to a novel dealkoxyphenylation reaction, and specifically, a method for obtaining a substrate having a hydroxy group from a substrate such as a sugar bound to a phenyl group substituted by alkoxy at the para-or ortho-position through an oxygen atom.

### Background Art

In the chemical synthesis of oligosaccharide chains etc., having (multiple) hydroxy groups, there is a need to rationally use techniques for selective protection and deprotection of such hydroxy groups in order to efficiently obtain a target compound. From this viewpoint, in the chemical synthesis of sugars etc., various techniques for using, protecting, and deprotecting protective groups have been developed. Among these, particularly, a para-methoxyphenyl group can be stably handled under both acidic and basic conditions, and can be easily oxidatively deprotected, and thus has been widely used as a general protective group at the sugar anomeric position, for example (Non Patent Literature 1).

A common and long-used technique for deprotecting the above para-methoxyphenyl group is a method using ammonium cerium (IV) nitrate (Non Patent Literature 2). The method provides target deprotected products with moderate to high yields for a wide range of substrates; however, the method generally requires the use of an excess amount of ammonium cerium (IV) nitrate, followed by a need of reduction treatment of an excess amount of an oxidizing agent during post-treatment, and has been reported to result in cases where a target deprotected product cannot always be obtained with a satisfactory yield depending on a substrate applied (Non Patent Literature 3), for example. For these reasons, the development of a more efficient technique has been desired.

As a means for achieving such an object, a method for deprotecting a para-methoxyphenyl group at the anomeric position by electrolytic reaction has been reported (Non Patent Literature 3), but the implementation of this method requires special experimental devices, and thus the method is considered to be a method that is difficult to be scaled up due to equipment constraints.

A method has also been reported, which involves protecting a hydroxy group at the anomeric position of a sugar with a para-methoxyphenyl group and then deprotecting the resultant in the presence of a zinc halide and an acid halide to convert it to a halogenated sugar (Non Patent Literature 4). However, it cannot be said that the method is performed under mild reaction conditions, since the benzyl group on the hydroxy group is converted to an acetyl group during deprotection, for example.

### Citation List

### Non Patent Literature

Non Patent Literature 1: Matsuzaki, Y.; Ito, Y.; Nakahara, Y.; Ogawa, T. Tetrahedron Lett. 1993, 34, 1061.
Non Patent Literature 2: Fukuyama, T.; Laird, A. A.; Hotchkiss, L. M. Tetrahedron Lett. 1985, 26, 6291.
Non Patent Literature 3: Iacobucci, S.; Filippova, N.; Alarcao, M. Carbohydrate Res. 1995, 277, 321.
Non Patent Literature 4: Zhang, Z.; Magnusson, G. Carbohydrate Res. 1996, 925, 41.

### Summary of Invention

### Technical Problem

From the above background, it has been desired to develop a de-para-methoxyphenylation reaction etc., by which the protective group such as a para-methoxyphenyl group can be deprotected under milder conditions and the deprotected product can be obtained with a high yield.

### Solution to Problem

The present inventors have discovered that a dealkoxyphenylation product can be obtained with a high yield under mild conditions by reacting λ3-iodane with (or causing the same to act on) a substrate bound to a phenyl group substituted by C₁ to C₅ alkoxy at the para- or ortho-position through an oxygen atom in a fluorous alcohol and water, and thus have completed the present invention.

Specifically, the present invention relates to the following:
[1] A method for producing a compound represented by the formula R-OH, comprising a step of reacting a compound represented by the formula R-OX with λ3-iodane in a fluorous alcohol and water, wherein R is a substrate, X is a phenyl group substituted by C₁ to C ₅ alkoxy at the para- or ortho-position, and the phenyl group may optionally be further substituted.
[2] The method according to [1], wherein the C₁ to C₅ alkoxy in the X group is methoxy, ethoxy, propyloxy, or isopropyloxy.
[3] The method according to [1], wherein the C₁ to C₅ alkoxy in the X group is paramethoxy.
[4] The method according to any one of [1] to [3], wherein the λ3-iodane is a compound represented by the formula R¹-I(OR²)₂, wherein R¹ is an unsubstituted or substituted phenyl group, and R² is selected from the group consisting of H, acetyl, trifluoroacetyl, tosyl, methanesulfonyl, and a combination thereof.
[5] The method according to [4], wherein the compound represented by the formula R¹-I(OR²)₂ is selected from the group consisting of [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, [hydroxy(methanesulfonyloxy)iodo]benzene, and a combination thereof.
[6] The method according to any one of [1] to [5], wherein the amount of the λ3-iodane is 0.1 to 10 equivalents relative to the substrate.
[7] The method according to any one of [1] to [6], wherein the fluorous alcohol is a fluorous aliphatic alcohol.
[8] The method according to [7], wherein the fluorous aliphatic alcohol is a fluorous C₂ to C₈ aliphatic alcohol.
[9] The method according to [8], wherein the fluorous C₂ to C₈ aliphatic alcohol is selected from the group consisting of hexafluoro 2-propanol (HFIP), 2,2,2-trifluoroethanol (TFE), 2,2,3,3,4,4,5,5-octafluoro-1-pentanol, nonafluoro-tert-butyl alcohol, and a combination thereof.
[10] The method according to any one of [1] to [9], comprising adding a solvent selected from the group consisting of CH₂Cl₂, toluene, (trifluoromethyl)benzene, and a combination thereof.
[11] The method according to any one of [1] to [10], wherein the amount of the fluorous alcohol is 1.0 equivalent or more in a molar ratio and 15 or less in a volume ratio relative to the substrate.
[12] The method according to any one of [1] to [11], wherein the amount of the water is 1.0 equivalent or more in a molar ratio and 10 or less in a volume ratio relative to the substrate.
[13] The method according to any one of [1] to [12], comprising adding an additive selected from the group consisting of sodium dihydrogen phosphate (NaH₂PO₄), potassium dihydrogen phosphate (KH₂PO₄), disodium hydrogen phosphate (Na₂HPO₄), and a combination thereof.
[14] The method according to any one of [5] to [13], comprising adding trifluoroacetic acid when (diacetoxyiodo)benzene (PIDA) is used as the λ3-iodane.
[15] The method according to any one of [1] to [14], which is performed at -20°C to 60°C.
[16] The method according to any one of [1] to [15], wherein the substrate R is a sugar having the OX group at the 1-position or the anomeric position.
[17] The method according to [16], wherein the sugar is a monosaccharide or a polysaccharide.
[18] The method according to [17], wherein the monosaccharide has a 5-membered ring or 6-membered ring structure.
[19] The method according to [18], wherein the monosaccharide is a pentose or a hexose.
[20] The method according to [19], wherein the hexose is glucose, mannose, galactose, or glucosamine.
[21] The method according to [17], wherein the polysaccharide is a disaccharide to a decasaccharide.
[22] The method according to [17], wherein the polysaccharide is (i) a disaccharide that is galactose-glucosamine, glucosamine-glucosamine, neuraminic acid-galactose, or mannose-glucosamine, (ii) a trisaccharide consisting of two mannoses and one glucosamine, or a trisaccharide consisting of neuraminic acid, galactose and glucosamine, or (iii) a tetrasaccharide consisting of two mannoses and two glucosamines, or a tetrasaccharide consisting of three mannoses and one glucosamine.
[23] The method according to any one of [16] to [22], wherein a hydroxy group of a carbon adjacent to the carbon at the 1-position or the anomeric position in the sugar is protected with an acyl group, or, an amino group of a carbon adjacent to the carbon at the 1-position or the anomeric position in the sugar is protected with an imide group, an acyl group, or a carbamate group, or a carbon adjacent to the carbon at the 1-position or anomeric position in the sugar has an azide group (N₃).
[24] The method according to [23], wherein the imide group as the amino group-protecting group is phthaloyl (Phth), the acyl group is acetyl group (Ac), and the carbamate group is selected from the group consisting of (2,2,2-trichloroethoxy)carbonyl (Troc), allyloxycarbonyl (Alloc), 2-(trimethylsilyl)ethoxycarbonyl (Teoc), 9-fluorenylmethyloxycarbonyl (Fmoc), tert-butoxycarbonyl (Boc), and benzyloxycarbonyl (Cbz).
[25] The method according to [23], wherein the acyl group as the hydroxy group-protecting group is selected from the group consisting of acetyl (Ac) and benzoyl (Bz).
[26] A method for producing a sugar to which one or two or more attaching moieties are bound, comprising a step of binding one or two or more attaching moieties to a sugar obtained by the method according to any one of [16] to [25], wherein the one or two or more attaching moieties are selected from the group consisting of a protein, a nucleic acid molecule, a lipid molecule, a eukaryotic cell, a prokaryotic cell, a tissue derived from an organism, a virus, a parasite, a low-molecular-weight compound, and an artifact.
[27] The method according to [26], wherein the attaching moiety is a protein.
[28] The method according to [27], wherein the protein is a receptor, and wherein the receptor is a soluble receptor, fused to the Fc region of an antibody, or unmodified.
[29] The method according to [27], wherein
   the protein is an antibody or an antigen-binding fragment thereof, and
   wherein the antibody or the antigen-binding fragment thereof is bound to a peptide, a nucleic acid molecule, a lipid molecule, a low-molecular-weight compound, an artifact, another antibody or an antigen-binding fragment thereof, or a toxin, or forms a complex with a drug, or is unmodified.
[30] The method according to [27], wherein the protein is a cytokine, and wherein the cytokine is bound to an antibody or an antigen-binding fragment thereof, or is unmodified.
[31] The method according to any one of [26] to [30], comprising a step of binding to the sugar one or two or more additional attaching moieties other than the attaching moiety that is a protein.
[32] The method according to any one of [1] to [15], wherein the substrate R is Ar-(CR¹R²)n-, wherein n = 1 to 3, Ar is an aromatic ring, R¹ and R² are each H, an aromatic ring, or an aliphatic group, and wherein any of the aromatic ring and the aliphatic group may be optionally substituted.
[33] The method according to [32], wherein the aromatic ring is a C₅ to C₂₀ aryl group or a 5- to 20-membered ring heteroaryl group that may be optionally substituted, and wherein the aliphatic group is a C₁ to C₁₀ aliphatic hydrocarbon group that may be optionally substituted.
[34] The method according to [32] or [33], wherein the aromatic ring is selected from the group consisting of xylene, toluene, styrene, ethylbenzene, cumene, furan, thiophene, pyrrole, pyran, thiopyran, pyridine, thiazole, imidazole, pyrimidine, 1,3,5-triazine, naphthalene, indene, anthracene, phenanthrene, fluorene, biphenyl, triphenyl, terphenyl, binaphthyl, phenyl naphthalene, indole, quinoline, and purine.

### Advantageous Effects of Invention

According to the present invention, a dealkoxyphenylation product can be obtained with a high yield from a substrate such as a sugar bound to a phenyl group substituted by C₁ to C₅ alkoxy at the para- or ortho-position through an oxygen atom under mild conditions. Therefore, the method according to the present invention is highly safe in operation, can be performed with a simple operation, enables easy purification of the product, and is suitable for large-scale synthesis of such a product.

### Description of Embodiments

The present invention provides a method for obtaining a compound represented by the formula R-OH, comprising reacting λ3-iodane in a fluorous alcohol and water with (or causing the same to act on) a compound represented by the formula R-OX, wherein R is a substrate, X is a phenyl group substituted by C₁ to C₅ alkoxy at the para- or ortho-position, and the phenyl group may be further optionally substituted.

In the present Description, a reaction in which a compound represented by the formula R-OH is produced from the compound represented by the formula R-OX is referred to as a "dealkoxyphenylation reaction." The above reaction mechanism is understood as the one, but is not limited thereto, by which λ3-iodane acts as a one-electron oxidant on the compound represented by the formula R-OX, the OX group is eliminated from the compound represented by the formula R-OX, and then water (H₂O) is added thereto, thereby producing the compound represented by the formula R-OH. In the field of synthetic organic chemistry including the chemical synthesis of sugar chains, the frequent use of para-methoxyphenyl groups and the like as "protective groups" for highly reactive functional groups such as hydroxy groups in order to achieve target reactions is well known to those skilled in the art. However, the present invention is not limited to the use for such deprotection purposes, and is applied to any case of producing the compound represented by the formula R-OH from the compound represented by the formula R-OX. In the present Description, in order to facilitate understanding, the compound represented by the formula R-OX may be referred to as a "protected product", and the alkoxyphenyl group (X group) may be referred to as "protective group", the dealkoxyphenylation reaction may be referred to as the "deprotection reaction", and the resulting compound represented by the formula R-OH may be referred to as the "deprotected product" for convenience.

"R" in the compound represented by the formula R-OX refers to a substrate. "Substrate" refers to any organic compound capable of binding to a phenyl group (X group) substituted by C₁ to C₅ alkoxy at the para- or ortho-position through an oxygen atom.

In one embodiment, the substrate R may be a "sugar". Sugars include monosaccharides and polysaccharides. The "monosaccharide" may be either a D-isomer or an L-isomer, or an aldose or a ketose.

In one embodiment, in the present invention, a polyhydric alcohol having a 5-membered ring or 6-membered ring structure wherein one of the carbons forming the ring structure is replaced by oxygen as the "monosaccharide", and two or more hydroxy groups are contained, can be suitably used. More specifically, monosaccharides such as a five-carbon sugar (pentose), a six-carbon sugar (hexose), a seven-carbon sugar (heptose), an eight-carbon sugar (octulose), and a nine-carbon sugar (nonose) that form five-membered ring or six-membered ring structures are suitably used, pentose and hexose are more preferably used, and hexose is even more preferably used. Specific examples of pentose include ribose, xylose, aviose, arabinose, ribulose, and xylulose. Specific examples of hexose include glucose, mannose, galactose, altrose, psicose, and fructose, and for example, glucose, mannose and galactose are suitably used.

In one embodiment, in the present invention, "polysaccharide" may be used as a substrate. The "polysaccharide" refers to a sugar in which two or more monosaccharides are linked by glycosidic bonds, and includes sugars generally referred to as oligosaccharides. In the present invention, the polysaccharide is preferably a polysaccharide composed of monosaccharides forming five- membered ring or six-membered ring structures as described above, and disaccharides to decasaccharides are suitably used, but examples thereof are not limited to these.

Further, in the present invention, examples of the "sugar" also include monosaccharide or polysaccharide derivatives. Examples of monosaccharide derivatives include amino sugars (glucosamine, galactosamine, mannosamine, neuraminic acid, sialic acid, muramic acid, etc.), deoxysugars (deoxyribose, deoxyglucose, rhamnose, fucose, etc.), and uronic acids (glucuronic acid, guluronic acid, mannuronic acid, galacturonic acid, iduronic acid, etc.), or derivatives thereof (e.g., acetylated derivatives). For example, the amino sugars or derivatives thereof (e.g., N-acetylglucosamine, N-acetylgalactosamine, *N*-acetylneuraminic acid, etc.) are suitably used. Moreover, a polysaccharide derivative refers to a sugar, in which two or more monosaccharides are bound together, and refers to the one containing at least one of the above monosaccharide derivatives.

Suitable examples of polysaccharides in the present invention include disaccharides, trisaccharides, or tetrasaccharides at any position constituting a human type N-type sialylglycopeptide (SGP), and specific examples thereof include (i) disaccharides such as galactose-glucosamine, glucosamine-glucosamine, neuraminic acid-galactose, and mannose-glucosamine, (ii) a trisaccharide consisting of two mannoses and one glucosamine, or a trisaccharide consisting of neuraminic acid, galactose and glucosamine, and (iii) a tetrasaccharide consisting of two mannoses and two glucosamines, or a tetrasaccharide consisting of three mannoses and one glucosamine. Another example of the polysaccharide in the present invention is a decasaccharide in which one N-acetylglucosamine (GlcNAc) at the reducing end of the sugar chain portion (SG) of SGP is deleted.

In one embodiment, other moieties (hereinafter referred to as "attaching moiety") may be bound to sugars (monosaccharides, polysaccharides, derivatives thereof, etc.) as substrates in the present invention.

In another embodiment, a sugar represented by the formula R-OH (hereinafter referred to as "resulted sugar") is produced by the method of the present invention from a sugar-containing compound represented by the formula R-OX, wherein the substrate R is the sugar, and then through a further reaction, another sugar is added to the resulted sugar or a protein and the like are added to the resulted sugar for modification, so that a sugar derivative (hereinafter referred to as a "resulted sugar derivative" or "derivative of resulted sugar") can be produced. The resulted sugar and the resulted sugar derivative may contain substances other than sugar, such as proteins, low-molecular-weight compounds, nucleic acid molecules, peptides, lipids, artifacts, etc., as "attaching moieties". The present invention provides a method for producing a resulted sugar and a method for producing a resulted sugar derivative. The resulted sugar and the resulted sugar derivative may have activities or functions etc., such as catalytic activity, labeling functions, functions of an enzyme substrate, cytotoxic activity, immunocyte-activating activity, antioxidant activity, protective action, receptor functions, ligand functions, pharmacokinetics regulating function, drug delivery function, and the like, due to the presence of an "attaching moiety" such as a protein.

Examples of the above "attaching moiety" to a sugar can include, but are not limited to, biopolymers such as proteins, nucleic acid molecules, and lipid molecules, cells (eukaryotic cells, prokaryotic cells), tissues derived from organisms, parasites such as viruses, low-molecular-weight compounds, and artifacts. The "attaching moiety" may have activities or functions etc., such as catalytic activity, labeling function, functions of an enzyme substrate, cytotoxic activity, immune cell activating activity, antigen binding activity, protective action, receptor function, ligand function, pharmacokinetics regulating function, drug delivery function, and infectivity. Examples of a protein as one aspect of the "attaching moiety" can include those derived from humans or non-human animals, wild-type ones, modified ones, and artificially designed ones, and preferred examples of the same can include cytokines, receptors, immunoglobulins (antibodies) or fragments thereof. Examples of suitable cytokines can include, but are not limited to, wild-type human cytokines, immunocytokines, and fragments thereof. Examples of suitable receptors can include, but are not limited to, soluble-form receptors, and nuclear receptors, and may be fusions of the receptor or a fragment thereof with an immunoglobulin Fc region. Antibodies may be full bodies, antigen-binding fragments of antibodies such as scFv, conjugates of antibodies or antigen-binding fragments thereof and drugs (hereinafter referred to as "antibody-drug conjugates"), multi-specific antibodies, immunotoxins, labeled antibodies or binding fragments thereof etc. In the present invention, they may also be referred to as "proteins". A "low-molecular-weight compound" as one aspect of the "attaching moiety" may be a platinum-based compound such as cisplatin and carboplatin. As described above, the present invention also provides a method for producing a resulted sugar to which an attaching moiety is bound, which comprises a step of binding the attaching moiety to the resulted sugar, and also a method for producing a resulted sugar derivative to which an attaching moiety is bound, which comprises a step of binding the attaching moiety to the resulted sugar derivative. Here, the resulted sugar and the resulted sugar derivative, to which an attaching moiety is bound, may be further modified or altered, and the modified or altered product may be included in the scope of "the resulted sugar, to which an attaching moiety is bound" and "a resulted sugar derivative, to which an attaching moiety is bound".

Further, the first attaching moiety and the second attaching moiety can be bound (linked) via the resulted sugar or resulted sugar derivative of the present invention. For example, an immunocytokine formed by binding of an antibody or an antigen-binding fragment thereof with a cytokine, an immunotoxin formed by binding of an antibody or an antigen-binding fragment thereof with a toxin, or an antibody-drug conjugate formed by binding of an antibody or an antigen-binding thereof with a drug can be produced through the resulted sugar or resulted sugar derivative. Therefore, the present invention also provides a method for producing a resulted sugar formed by binding the first attaching moiety and the second attaching moiety, and a method for producing a resulted sugar derivative formed by binding the first attaching moiety and the second attaching moiety. Examples of a combination of the first attaching moiety and the second attaching moiety can include, but are not limited to, a first antibody and a second antibody, an antibody and a cytokine, an antibody and a toxin, an antibody and a drug, and a receptor fragment and the Fc region etc., of an antibody. In another aspect, the attaching moiety may be bound to or contained in a compound represented by the formula R-OX or a compound represented by the formula R-OH. Moreover, the compound represented by the formula R-OH containing the attaching moiety can be referred to as "attaching moiety-containing product". From the compound represented by the formula R-OX, wherein R is a substrate containing the attaching moiety, the attaching moiety-containing product can be produced by the methods provided by the present invention.

In the present invention, when a sugar is the substrate, the above X group (a phenyl group substituted by C₁ to C₅ alkoxy at the para- or ortho-position) is covalently bound to the hydroxy group present in the carbon at the 1-position or anomeric position in the sugar, forming an "OX group".

In the present invention, in the substrate sugar, the hydroxy group of the carbon adjacent to the carbon at the 1-position or anomeric position having the OX group may be protected with an acyl group such as acetyl (Ac) or benzoyl (Bz), an ether-based protective group such as benzyl (Bn), or a silyl-based protective group such as trimethylsilyl (TMS), tert-butyldimethylsilyl (TBS), tert-butyldiphenylsilyl (TBDPS), or triisopropylsilyl (TIPS). Alternatively, in the substrate sugar, the amino group of the carbon adjacent to the carbon at the 1-position or anomeric position having the OX group (for example, when a sugar has an amino group as in the case of an amino sugar) may be protected with an imide group such as phthaloyl (Phth), an acyl group such as acetyl (Ac), or a carbamate group such as (2,2,2-trichloroethoxy)carbonyl (Troc), allyloxycarbonyl (Alloc), 2-(trimethylsilyl)ethoxycarbonyl (Teoc), 9-fluorenylmethyloxycarbonyl (Fmoc), tert-butoxycarbonyl (Boc), or benzyloxycarbonyl (Cbz). Alternatively, in the substrate sugar, the carbon adjacent to the carbon at the 1-position or anomeric position having the OX group may have an azide group (N₃). In particular, in the substrate sugar, the hydroxy group of the carbon adjacent to the carbon at the 1-position or anomeric position having the OX group is preferably protected with an acyl group, or an amino group of the carbon adjacent to the carbon at the 1-position or anomeric position having the OX group is preferably protected with an imide group or a carbamate group. As described above, the presence of a protective group having a carbonyl oxygen atom for the hydroxy group or the amino group on the carbon adjacent to the carbon at the 1-position or anomeric position having the OX group is very useful in increasing the yield of the dealkoxyphenylation reaction product. Other hydroxy groups present in the sugar may be unsubstituted and protected with an acyl group (e.g., acetyl (Ac) and benzoyl (Bz)), an ether group (e.g., benzyl (Bn), 2-naphthyl methyl (Nap), methoxymethyl (MOM), dihydropyran (DHP) and allyl), a silyl group (e.g., trimethylsilyl (TMS) tert-butyldimethylsilyl (TBS) and TBDPS (tert-butyldiphenylsilyl)), or a trityl group (e.g., triphenylmethyl) or the like.

In another embodiment, the substrate R may be Ar-(CR¹R²)n-, wherein n = 1 to 3, preferably n = 1 to 2, particularly preferably n = 1, Ar is an aromatic ring, R¹ and R² are each H, an aromatic ring, or an aliphatic group, and any of the aromatic ring and the aliphatic group may be optionally substituted.

Examples of the "aromatic ring" defined for Ar, R¹ and R² in Ar-(CR¹R²)n-set forth above can include a monocyclic aromatic ring and a polycyclic aromatic ring. Examples of the "monocyclic aromatic ring" can include a monocyclic aromatic hydrocarbon or a monocyclic heteroaromatic ring (heteroaromatic ring refers to a heterocyclic compound having aromaticity). Examples of the "monocyclic aromatic hydrocarbon" include an unsubstituted benzene. As will be described later, the benzene may be substituted, and specific examples of benzene having a hydrocarbon substituent include xylene, toluene, styrene, ethylbenzene, and cumene. Examples of the "monocyclic heteroaromatic ring" can include furan, thiophene, pyrrole, pyran, thiopyran, pyridine, thiazole, imidazole, pyrimidine, and 1,3,5-triazine. Examples of the "polycyclic aromatic ring" can include a polycyclic aromatic hydrocarbon and polycyclic heteroaromatic rings. Examples of the "polycyclic aromatic hydrocarbon" include a ring-assembled aromatic hydrocarbon (that is, an aromatic compound in which two or more aromatic rings, for example, 2 to 5 aromatic rings are directly linked), and a condensed polycyclic aromatic hydrocarbon (a compound in which two or more aromatic rings, such as 2 to 5 aromatic rings are condensed), and can include naphthalene, indene, anthracene, phenanthrene, fluorene, biphenyl, triphenyl, terphenyl, binaphthyl, and phenylnaphthalene. Examples of the "polycyclic heteroaromatic rings" include ring-assembled heteroaromatic rings (a compound having at least one heteroaromatic ring, in which two or more, for example, 2 to 5 aromatic rings or heteroaromatic rings are directly linked) and condensed polycyclic heteroaromatic rings (a compound having at least one heteroaromatic ring, in which two or more, for example 2 to 5 aromatic or heteroaromatic rings are condensed), and can include indole, quinoline, and purine. The above aromatic ring may also be represented as an aryl or heteroaryl group, and may also be a C₅ to C₂₀ aryl group, a C₅ to C ₁₄ aryl group, or a C₅ to C₁₀ aryl group, or, a 5- to 20-membered ring heteroaryl group, a 5-to 14-membered ring heteroaryl group, or a 5- to 10-membered ring heteroaryl group, but examples thereof are not limited thereto. The above "aromatic ring" may be unsubstituted or may have one or more substituents, and examples of the substituents include linear or branched saturated or unsaturated hydrocarbon groups, oxygen-containing groups (hydroxy, alkoxy, aldehyde, ketone, acetoxy, acetyl, carbonyl, oxy, carboxyl, ester, etc.), nitrogen-containing groups (amino, cyano, imide, azo, azide, etc.), sulfur-containing groups (sulfonyl, thiol, etc.) and halogens (e.g., fluorine, chlorine, bromine, and iodine), and more preferable examples thereof are hydrocarbon groups, oxygen-containing groups, and halogens. When these substituents contain carbons, for example, those having 1 to 10 carbons, those having 1 to 5 carbons, or those having 1 to 3 carbons can be suitably used (e.g., C₁ to C₁₀ hydrocarbon group, C₁ to C₅ hydrocarbon group, or C₁ to C₃ hydrocarbon group, or, C₁ to C₁₀ alkoxy, C₁ to C₅ alkoxy, or C₁ to C₃ alkoxy, etc., can be suitably used).

Examples of the "aliphatic group" defined for R¹ and R² in Ar-(CR¹R²)n- set forth above include a C₁ to C₁₀ hydrocarbon group, preferably a C₁ to C₅ hydrocarbon group, and more preferably a C₁ to C₃ hydrocarbon group, and may be saturated or unsaturated acyclic, or saturated or unsaturated cyclic. Also, when the hydrocarbon group is acyclic, it may be linear or branched. Examples of the "C₁ to C₁₀ hydrocarbon group" include a C₁ to C₁₀ alkyl group, a C₂ to C₁₀ alkenyl group, a C₂ to C₁₀ alkynyl group, a C₄ to C₁₀ alkyldienyl group, a C₄ to C₁₀ cycloalkyl group, and a C₄ to C₁₀ cycloalkenyl group, and may also be a C₁ to C₆ hydrocarbon group or a C₁ to C₃ hydrocarbon group. Examples of the C₁ to C₁₀ alkyl group include methyl, ethyl, propyl, isopropyl, n-butyl, sec-butyl, tert-butyl, pentyl, hexyl, octyl, nonyl, and decyl. Examples of the C₂ to C₁₀ alkenyl group include vinyl, allyl, propenyl, isopropenyl, 2-methyl-1-propenyl, and 2-butenyl. Examples of the C₂ to C₁₀ alkynyl include ethynyl, 2-propynyl, and 2-butynyl. Examples of the C₄ to C₁₀ alkyldienyl group include 1,3-butadienyl. Examples of the C₄ to C₁₀ cycloalkyl group include cyclopropyl, cyclobutyl, cyclopentyl, and cyclohexyl. Examples of the C₄ to C₁₀ cycloalkenyl group include cyclopropenyl, cyclobutenyl, 2-cyclopenten-1-yl, and 2-cyclohexen-1-yl. Note that the examples of the above hydrocarbon groups are referred to throughout the present Description. Further, the above "aliphatic group" may be unsubstituted or may have one or more substituents, wherein at least one hydrogen atom in a group such as the C₁ to C₁₀ hydrocarbon group may be substituted by, a non-hydrogen atom or group such as an oxygen-containing group (hydroxy, alkoxy, aldehyde, ketone, acetoxy, acetyl, carbonyl, oxy, carboxyl, ester, etc.), a nitrogen-containing group (amino, cyano, isocyanate, imide, azo, azide, etc.), a sulfur-containing group (sulfonyl, thiol, etc.), and a halogen (e.g., fluorine, chlorine, bromine, iodine), or, may have or contain these groups, and a preferable example thereof is an oxygen-containing group or halogen. Furthermore, the above "aliphatic group" may be substituted by a C₅ to C₂₀ aryl group (e.g., a C₅ to C₁₄ aryl group or a C₅ to C₁₀ aryl group).

"OX" in the above formula R-OX indicates that oxygen (O) and X are covalently bound, and "X" represents a phenyl group substituted by C₁ to C₅ alkoxy (may also be referred to as alkyloxy group) at the para- or ortho-position. In a compound represented by the formula R-OX, one OX group may be present or a plurality of OX groups may be present. Specific examples of the above "C₁ to C₅ alkoxy" include methoxy, ethoxy, propyloxy, 1-methylethoxy, butoxy, 2-methyl propyloxy, 1-methyl propyloxy, 1,1-dimethylethoxy, pentyloxy groups, 3-methylbutoxy, 2-methylbutoxy, 2,2-dimethyl propyloxy, 1-ethyl propyloxy, and 1,1-dimethyl propyloxy, and C₁ to C₃ alkoxy is preferably methoxy, ethoxy, propyloxy, and isopropyloxy, for example, more preferably methoxy and ethoxy, and even more preferably methoxy. The above examples of "C₁ to C₅ alkoxy" are also referred to throughout the present Description. Further, the above "C₁ to C₅ alkoxy" may be positioned at either the para-position or the ortho-position of a phenyl group, but the para-position is preferred. Moreover, the above phenyl group in the X group may be unsubstituted or further optionally substituted with one or more substituents. Examples of the substituents include a linear or branched saturated or unsaturated hydrocarbon group, an oxygen-containing group (hydroxy, alkoxy, aldehyde, ketone, acetoxy, acetyl, carboxyl, ester, etc.), a nitrogen-containing group (amino, cyano, imide, azo, azide, etc.), a sulfur-containing group (sulfonyl, thiol, etc.), a halogen (e.g., fluorine, chlorine, bromine, and iodine), and more preferable examples thereof include hydrocarbon groups, oxygen-containing substituents, and halogens. When these substituents contain carbons, for example, those having 1 to 10 carbons, those having 1 to 5 carbons, or those having 1 to 3 carbons can be suitably used (e.g., C₁ to C₁₀ hydrocarbon group, C₁ to C₅ hydrocarbon group, or C₁ to C₃ hydrocarbon group, or, C₁ to C₁₀ alkoxy, C₁ to C₅ alkoxy, or C₁ to C₃ alkoxy, etc., can be suitably used).

"λ3-iodane" refers to a trivalent hypervalent iodine compound. The use of λ3-iodane results in higher yields of dealkoxyphenylation reaction products and can be performed under milder reaction conditions than those of conventional deprotection methods. Further, the reaction proceeds with a small excess of λ3-iodane, so as to facilitate the purification of the deprotected product compared with the conventional deprotection methods using, for example, an excess amount of ammonium cerium (IV) nitrate, and to result in high operational safety.

In one embodiment, λ3-iodane is a compound represented by the formula R¹-I(OR²)₂ (wherein R¹ is an unsubstituted or substituted phenyl group, R² is selected from the group consisting of H, acetoxy, trifluoroacetoxy, tosyloxy, methanesulfonyloxy, and a combination thereof). As defined in the above formula, R¹ may be a "substituted phenyl group", and examples of the substituent include linear or branched saturated or unsaturated hydrocarbon groups, oxygen-containing groups (alkoxy, ester etc.), nitrogen-containing groups (cyano, azide, etc.), and halogens (e.g., fluorine, chlorine, bromine, and iodine), and are more preferably hydrocarbon groups, oxygen-containing substituents, and halogens. When these substituents contain carbons, for example, those having 1 to 5 carbons or those having 1 to 3 carbons can be suitably used. Specific examples of λ3-iodane include, but are not limited to, [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, and [hydroxy(methanesulfonyloxy)iodo]benzene.

The amount of λ3-iodane can be appropriately set from the viewpoint etc., of achieving a high yield of the product depending on the type of a substrate, and can be about 0.1 to 10 equivalents, about 0.5 to 7 equivalents, or about 1 to 5 equivalents, for example, relative to the substrate. These amounts are suitably applied to any substrate, and particularly when the substrate is a monosaccharide, the amount is more preferably about 1 to 3 equivalents, when the substrate is a disaccharide, the amount is more preferably about 1 to 4.5 equivalents, and when the substrate is Ar-(CR¹R²)n- above, the amount is more preferably about 1 to 3 equivalents. It should be noted that throughout the present Description, the term "about" indicates a range of ±10% of the stated value.

"Fluorous alcohol" refers to a fluorine-containing alcohol compound in which all carbons have fluorine(s) except the carbons bound to the alcohol. As long as fluorine substitution is allowed, the fluorous alcohol preferably has more fluorines. Examples of the fluorous alcohol include, but are not limited to, fluorous aliphatic alcohols. The hydrocarbon moiety in the fluorous aliphatic alcohol may be saturated or unsaturated, linear or branched, or cyclic. The fluorous aliphatic alcohol is, for example, a fluorous C₂ to C₈ aliphatic alcohol, preferably a fluorous C₂ to C₅ aliphatic alcohol, more preferably a fluorous C₂ to C₃ aliphatic alcohol. Specific examples of the fluorous alcohol include, but are not limited to, hexafluoro 2-propanol (HFIP), 2,2,2-trifluoroethanol (TFE), 2,2,3,3,4,4,5,5-octafluoro-1-pentanol, nonafluoro-tert-butyl alcohol, and a combination thereof.

Furthermore, a deprotected product can be obtained with a higher yield with the use of a preferred combination of a fluorous alcohol and λ3-iodane. Such a combination can be appropriately selected by those skilled in the art. For example, as described in the Examples below, PIFA is suitably used in combination with hexafluoro 2-propanol (HFIP), 2,2,2-trifluoroethanol (TFE), nonafluoro-tert-butyl alcohol, etc., HTIB is suitably used in combination with HFIP, TFE, 2,2,3,3,4,4,5,5-octafluoro-1-pentanol etc., [bis(trifluoroacetoxy)iodo]pentafluorobenzene is suitably used in combination with hexafluoro 2-propanol (HFIP) etc., [hydroxy(methanesulfonyloxy)iodo]benzene is suitably used in combination with hexafluoro 2-propanol (HFIP), etc., but examples thereof are not limited to these examples of combination.

The amount of the fluorous alcohol can be appropriately set from the viewpoint etc., of achieving a high yield of the product. The amount can be about 1.0 equivalent or more, about 1.5 equivalents or more, about 2.0 equivalents or more, or 2.5 equivalents or more in a molar ratio relative to the substrate, and can be about 15 or less, about 10 or less, about 8 or less, or about 5 or less in a volume ratio relative to the substrate.

The dealkoxyphenylation reaction defined in the present invention is performed in the coexistence of the fluorous alcohol and "water". The amount of water can be appropriately set from the viewpoint etc., of achieving a high yield of the product. For example, the amount of water can be about 1.0 equivalent or more, about 1.5 equivalents or more, about 2.0 equivalents or more, or about 2.5 equivalents or more in a molar ratio relative to the substrate, and can be about 10 or less, about 8 or less, about 5 or less, or about 3 or less in a volume ratio relative to the substrate.

In the present invention, a "solvent" (also referred to as a reaction solvent) may further be added to the fluorous alcohol and water. The solvent can be selected from the group consisting of, but are not limited to, dichloromethane (CH₂Cl₂), toluene, (trifluoromethyl)benzene, and a combination thereof. The type of a solvent to be used can be appropriately selected according to the solubility of the substrate, the λ3-iodane etc., to be used, in order to achieve a high yield of the product. The amount of the solvent can also be set appropriately to achieve a high yield of the product, and can be about 0.5 to 50, about 1 to 20, or about 2 to 10 in a volume ratio relative to the substrate, for example.

In the present invention, an "additive" may further be added to the fluorous alcohol and water. The additive is preferably selected from the group consisting of sodium dihydrogen phosphate (NaH₂PO₄), potassium dihydrogen phosphate (KH₂PO₄), disodium hydrogen phosphate (Na₂HPO₄), and a combination thereof. As the dealkoxyphenylation reaction proceeds, the acidity can become too strong. Thus, particularly when λ3-iodane (HTIB etc.), which produces strongly acidic acid as a by-product, or a substrate that is susceptible to acidic conditions is used, the above additive such as sodium dihydrogen phosphate (NaH₂PO₄) is added, so that a higher product yield can be obtained. The amount of the additive can also be set appropriately to achieve a high yield of the product, and the amount of the additive can be about 0.5 to 8 equivalents, about 1 to 6 equivalents, or about 1.5 to 5 equivalents relative to the substrate, for example.

Further, when (diacetoxyiodo)benzene (PIDA) is used as λ3-iodane, trifluoroacetic acid (TFA) is preferably added in order to obtain a deprotected product with a higher yield.

As described above, in the present invention, in the coexistence of fluorous alcohol and water, λ3-iodane acts as a one-electron oxidant on phenyl (X group) having alkoxy at the ortho- or para-position in the formula R-OX, so that the OX group is eliminated from the compound represented by the formula R-OX, followed by the addition of water (H₂O) to it. As a result, it is considered that the action is achieved by making the alkoxyphenoxy group (OX group) easier to leave from the compound represented by the formula R-OX. Such action of λ3-iodane is generally achieved by stirring, refluxing, etc., a solution containing the compound represented by the formula R-OX and λ3-iodane in fluorous alcohol and water. Therefore, the method of the present invention can be conveniently implemented and scaling up thereof can be relatively easily performed. The dealkoxyphenylation product can be purified or isolated by any purification method known to those skilled in the art, such as crystallization of the product, and chromatography.

The temperature for λ3-iodane to react with a compound represented by the formula R-OX ranges from preferably about -20°C to the boiling point of the fluorous alcohol (for example, the boiling point of hexafluoro 2-propanol (HFIP) is about 58°C, and the same of 2,2,2-trifluoroethanol (TFE) is about 78°C) or lower, and can range from, for example, about -20°C to 60°C, about 0°C to 60°C, or about 10°C to 30°C. Further, since the dealkoxyphenylation reaction proceeds even at room temperature (15°C to 30°C), it is advantageous because there is no need of cooling or heating, and it is also effective when a heat-sensitive substrate is used.

Furthermore, the reaction time can be appropriately set in order to obtain a product with a high yield.

The present invention is more specifically illustrated in the following Examples, but these Examples do not limit the scope of the present invention.

### Examples

### <Example 1>

### (Example 1-1)

### Production and isolation of 4-O-acetyl-3,6-di-O-benzyl-2-deoxy-2-{ [(2,2,2-trichloroethoxy)carbonyl]amino}-D-glucopyranoside (2) by de-para-methoxyphenylation reaction

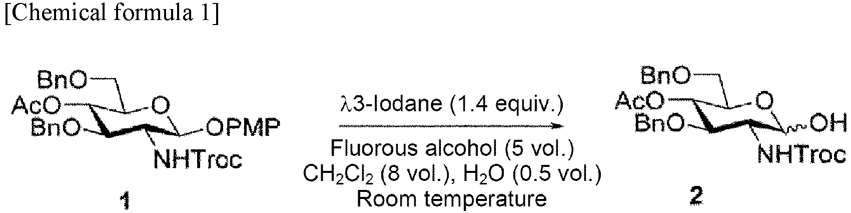

To a solution containing 4-methoxyphenyl 4-O-acetyl-3,6-di-O-benzyl-2-deoxy-2-{[(2,2,2-trichloroethoxy)carbonyl]amino}-β-D-glucopyranoside (1) (10.0 g, 14.64 mmol), dichloromethane (80 mL, 8 vol.), hexafluoro 2-propanol (HFIP) (50 mL, 5 vol.) and water (5 mL, 0.5 vol.), [bis(trifluoroacetoxy)iodo]benzene (PIFA) (8.82 g, 20.51 mmol, 1.4 equiv.) was added as λ3-iodane at room temperature (25°C or lower), and then the solution was stirred at the same temperature for 4 hours, thereby performing a de-para-methoxyphenylation reaction. After confirming the completion of the reaction by HPLC, ethyl acetate (250 mL) was added, the resultant was cooled with ice, water (100 mL) in which sodium hydrogen carbonate (5 g) and sodium sulfite (5 g) had been dissolved was poured, and then the resultant was subjected to liquid separation to obtain an organic layer. The obtained organic layer was washed again with water (100 mL) in which sodium hydrogen carbonate (5 g) and sodium sulfite (5 g) had been dissolved, and further washed with 20% saline (50 mL). The obtained organic layer was concentrated under reduced pressure in such a manner that the amount was 100 mL (precipitation of crystals was confirmed during concentration), and heptane (150 mL) was added dropwise. After cooling the obtained slurry liquid to 0°C to 5°C, the mixture was stirred at the same temperature for 1 hour, and then the precipitated crystals were filtered. The filtered crystals were washed with a mixture (8/24 mL) of ethyl acetate and heptane at 0°C to 5°C and dried under reduced pressure at 40°C, so that 4-O-acetyl-3,6-di-O-benzyl-2-deoxy-2-{[(2,2,2-trichloroethoxy)carbonyl]amino}-D-glucopyranoside (2) (7.6 g, 90% isolated yield) was isolated as white crystals. As described above, the yield of the deprotected product obtained by isolation after the de-para-methoxyphenylation reaction can be referred to as "isolated yield".

¹H-NMR (500 MHz, CDCl₃) δ 7.38-7.21 (m, 10H), 5.17 (t, J = 3.5 Hz, 1H), 5.13 (d, J = 10.0 Hz, 1H), 4.98 (t, J = 10.0 Hz, 1H) .4.78 (d, J = 12.0 Hz, 1H), 4.65-4.55 (m, 3H), 4.50 (dd, J = 17.2, 12.0 Hz, 2H), 4.41 (d, J = 2.9 Hz, 1H), 4.13-4.09 (m, 1H), 3.97 (td, J = 10.2, 2.5 Hz, 1H), 3.72 (t, J = 10.0 Hz, 1H), 3.51 (dd, J = 10.6, 7.2 Hz, 1H), 3.44 (dd, J = 10.0, 2.5 Hz, 1H), 1.90 (3H, s) .

¹³C-NMR (125 MHz, CDCl₃) δ 169.6, 154.1, 137.8, 137.2, 128.4, 128.1, 127.9, 127.7, 127.7, 95.3, 91.7, 77.3, 74.6, 73.7, 73.5, 70.9, 69.4, 68.8, 54.6, 20.7.

HRMS(ESI⁻)[M-H]⁻ [C₂₅H₂₇Cl₃NO₈] ⁻

Calculated value: 574.0808; Measured value 574.0834.

Yield measurements by HPLC analysis were performed under the following analytical conditions. In this Example 1 and Examples 2 to 14 described later, HPLC measurement was performed under the same analytical conditions.

### <HPLC analytical conditions>

Equipment used: SHIMAZU HPLC (2010A HT)
Column: Xbrige C18 3.5 µm, 4.6x150 mm (Waters)
Mobile phase A: 10 mM AcONH₄ aqueous solution
Mobile phase B: CH₃CN
Gradient conditions:

**[Table 1]**

| Time/solvent | 10 mM AcONH₄ aqueous solution | CH₃CN |
|---|---|---|
| 0 min | 85% | 15% |
| 7. 5 min | 50% | 50% |
| 10.5 min | 50% | 50% |
| 18.0 min | 5% | 95% |
| 23.0 min | 5% | 95% |

| | | |
|---|---|---|
| Flow rate: 1 mL/min. Detection wavelength: 210 nm Column temperature:40°C Injection volume: 5 µL Retention time: 18.3 minutes (1), 15.6 and 16.4 minutes (2), 14.7 minutes (Iodobenzene) | | |

### (Example 1-2)

### Production of 4-O-acetyl-3,6-di-O-benzyl-2-deoxy-2-{ [(2,2,2-trichloroethoxy)carbonyl]amino}-D-glucopyranoside (2) by de-para-methoxyphenylation reaction

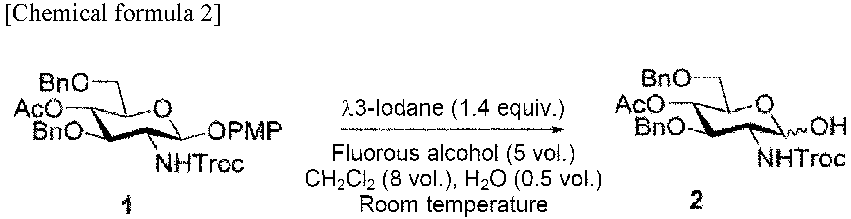

To a solution containing 4-methoxyphenyl 4-O-acetyl-3,6-di-O-benzyl-2-deoxy-2-{[(2,2,2-trichloroethoxy)carbonyl]amino}-β-D-glucopyranoside (1) (100 mg, 0.146 mmol), dichloromethane (0.8 mL, 8 vol.), hexafluoro 2-propanol (HFIP) (0.5 mL, 5 vol.) and water (0.05 mL, 0.5 vol.), [bis(trifluoroacetoxy)iodo]benzene (PIFA) (0.09 g, 0.205 mmol, 1.4 equiv.) was added as λ3-iodane at room temperature (25°C or lower), and then the solution was stirred at the same temperature for 2 hours, thereby performing a de-para-methoxyphenylation reaction. This solution was quantified by HPLC to calculate the yield (HPLC quantitative yield: 95%). In addition, as described above, a yield calculated after quantification by HPLC of the solution resulting from the de-para-methoxyphenylation reaction can be referred to as "HPLC quantitative yield".

### (Example 1-3)

Substrate 1 was subjected to a de-para-methoxyphenylation reaction performed by the same technique as in the method described in Example 1-2 using λ3-iodane, fluorous alcohol, and reaction time shown in Table 1 below. The solution after the reaction was quantified by HPLC to calculate the yield of product 2 (entries 2 to 7 in Table 1) (entry 1 in Table 1 relates to Example 1 (Example 1-1 (90%, isolation) and Example 1-2 (95%))).

### (Example 1-4)

To a solution containing 4-methoxyphenyl 4-O-acetyl-3,6-di-O-benzyl-2-deoxy-2-{[(2,2,2-trichloroethoxy)carbonyl]amino}-β-D-glucopyranoside (1) (101 mg, 0.148 mmol), trifluoroacetic acid (57 µL, 0.740 mmol, 5 equiv.), dichloromethane (1.0 mL, 10 vol.), hexafluoro 2-propanol (HFIP) (0.6 mL, 6 vol.) and water (0.05 mL, 0.5 vol.), (diacetoxyiodo)benzene (PIDA) (72 mg, 0.222 mmol, 1.4 equiv.) was added as λ3-iodane at room temperature (25°C or lower), and then the solution was stirred at the same temperature for 3 hours, thereby performing a de-para-methoxyphenylation reaction. This solution was quantified by HPLC and then the yield of 4-O-acetyl-3,6-di-O-benzyl-2-deoxy-2- { [(2,2,2-trichloroethoxy)carbonyl]amino}-D-glucopyranoside (2) was calculated (HPLC quantitative yield: 93%, entry 8 in Table 1).

**[Table 2]**

| Table 1 Results of de-para-methoxyphenylation reaction using various types of λ3-iodane and fluorous alcohol | | | | |
|---|---|---|---|---|
| Entry | λ3-iodane | Fluorous alcohol | Reaction time | Yield |
| 1 | PIFA | HFIP | 2h | 95% (90% (isolation)) |
| 2 | PIFA | 6^{d)} | 22h | 84% |
| 3 | HTIB | HFIP | 0.5h | 98% |
| 4 | 3al | HFIP | 24h | 86% |
| 5 | 4^{b)} | HFIP | 7h | >99% |
| 6 | HTIB | TFE | 4h | 94% |
| 7 | HTIB | 5^{c)} | 7h | 83% |
| 8 | PIDA | HFIP | 3h | 93% |

| | | | | |
|---|---|---|---|---|
| ^{a)} Bis(trifluoroacetoxy)iodo]pentafluorobenzene ^{b)} [Hydroxy(methanesulfonyloxy)iodo]benzene ^{c)} 2,2,3,3,4,4,5,5-Octafluoro-1-pentanol ^{d)} Nonafluoro-tert-butyl alcohol | | | | |

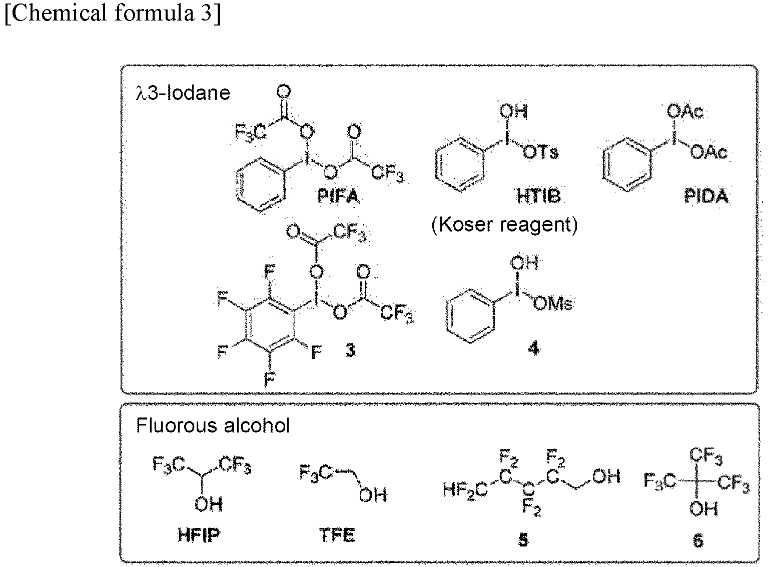

### <Example 2>

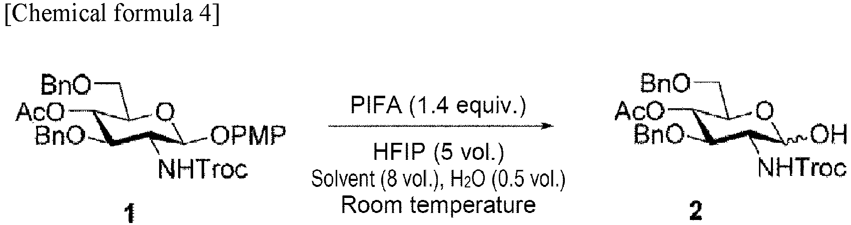

4-Methoxyphenyl 4-O-acetyl-3,6-di-O-benzyl-2-deoxy-2-{[(2,2,2-trichloroethoxy)carbonyl]amino}-β-D-glucopyranoside (1) was subjected to a de-para-methoxyphenylation reaction performed by the same technique as in the method described in Example 1-2 using various solvents and reaction times shown in Table 2. However, in entry 4 shown in Table 2 below, HTIB was used instead of PIFA. The solution after the reaction was quantified by HPLC, and then the yield of 4-O-acetyl-3,6-di-O-benzyl-2-deoxy-2-{[(2,2,2-trichloroethoxy)carbonyl]amino}-D-glucopyranoside (2) was calculated. The results are shown in Table 2 below.

**[Table 3]**

| Table 2 Results of de-para-methoxyphenylation reaction using various solvents | | | |
|---|---|---|---|
| Entry | Solvent | Time | Yield^{a)} |
| 1 | CH₂Cl₂ | 2h | 95% |
| 2 | Toluene | 23h | 94% |
| 3 | CF₃Ph | 2h | >99% |
| 4 | Toluene | 0.5h | >99% |

### <Example 3>

### Production of 4-O-acetyl-3,6-di-O-benzyl-2-deoxy-2-{ [(2,2,2-trichloroethoxy)carbonyl]amino}-D-glucopyranoside (2) by de-para-methoxyphenylation reaction performed using various amounts of λ3-iodane, fluorous alcohol, and water

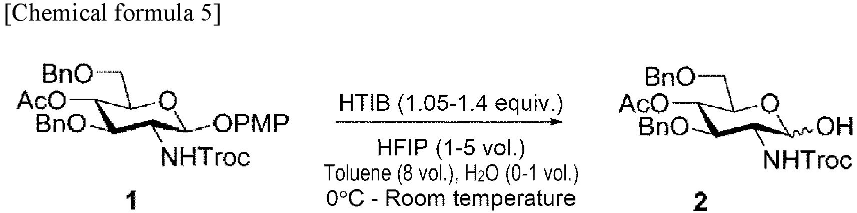

To a solution containing 4-methoxyphenyl 4-O-acetyl-3,6-di-O-benzyl-2-deoxy-2- { [(2,2,2-trichloroethoxy)carbonyl]amino}-β-D-glucopyranoside (1) (100 mg, 0.146 mmol), toluene (0.8 mL, 8 vol.), hexafluoro 2-propanol (HFIP) (0.5 mL, 5 vol.) and water (0.05 mL, 0.5 vol.), [hydroxy(tosyloxy)iodo]benzene (HTIB) (0.08 g, 0.205 mmol, 1.4 equiv.) was added as λ3-iodane at room temperature (25°C or lower), and then the solution was stirred at the same temperature for 0.5 hours.
This solution was quantified by HPLC, and then the yield of the obtained 4-O-acetyl-3,6-di-O-benzyl-2-deoxy-2-{[(2,2,2-trichloroethoxy)carbonyl]amino}-β-D-glucopyranoside (2) was calculated (entry 1, HPLC quantitative yield: >99%).

Substrate 1 was subjected to a de-para-methoxyphenylation reaction performed by the same technique as described above using λ3-iodane, fluorous alcohol, water, temperature, and reaction time shown in Table 3 below. The post-reaction solution was quantified by HPLC and then the yield of product 2 was calculated (entries 2 to 7). Entry 7 in Table 3 is an example of containing no water, but several impurities were significantly observed by HPLC and the yield significantly decreased. On the other hand, when water was present relative to the substrate, the product (deprotected product) was obtained with a high yield.

**[Table 4]**

| Table 3 Results of de-para-methoxyphenylation reaction performed using various amounts of HTIB, HFIP, and water | | | | | | |
|---|---|---|---|---|---|---|
| Entry | HTIB | HFIP | Water | Temperature | Time | Yield |
| 1 | 1.4 equiv. | 5 vol. | 0.5 vol. | Room temperature | 0.5h | >99% |
| 2 | 1.2 equiv. | 5 vol. | 0.5 vol. | Room temperature | 24h | 90% |
| 3 | 1.05 equiv. | 5 vol. | 0.5 vol. | Room temperature | 24h | 85% |
| 4 | 1.4 equiv. | 3 vol. | 0.5 vol. | Room temperature | 5h | 99% |
| 5 | 1.4 equiv. | 5 vol. | 1 vol. | Room temperature | 22h | 98% |
| 6 | 1.4 equiv. | 5 vol. | 0.5 vol. | 5°C | 4h | >99% |
| 7 | 1.4 equiv. | 5 vol. | 0 vol. | Room temperature | 3h | 37% |

### <Example 4>

### Production of 4-O-acetyl-3,6-di-O-benzyl-2-deoxy-2-{ [(2,2,2-trichloroethoxy)carbonyl]amino}-D-glucopyranoside (2) by de-para-methoxyphenylation reaction using various additives

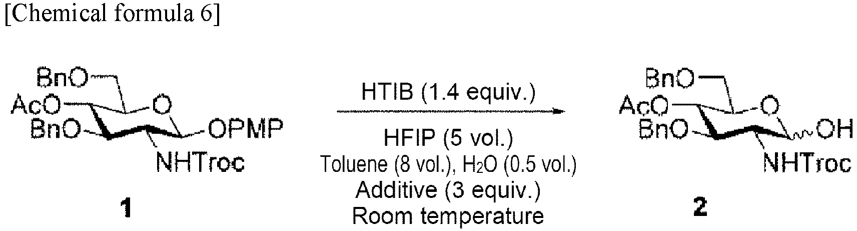

To a solution containing 4-methoxyphenyl 4-O-acetyl-3,6-di-O-benzyl-2-deoxy-2- { [(2,2,2-trichloroethoxy)carbonyl]amino}-β-D-glucopyranoside (1) (100 mg, 0.146 mmol), toluene (0.8 mL, 8 vol.), hexafluoro 2-propanol (HFIP) (0.5 mL, 5 vol.) and water (0.05 mL, 0.5 vol.), sodium dihydrogen phosphate (NaH₂PO₄) (0.05 g, 0.439 mmol, 3 equiv.) was added as an additive. Subsequently at room temperature (25°C or lower), [hydroxy(tosyloxy)iodo]benzene (HTIB) (0.08 g, 0.205 mmol, 1.4 equiv.) was added and then the solution was stirred for 2 hours at the same temperature. This solution was quantified by HPLC and then the yield of 4-O-acetyl-3,6-di-O-benzyl-2-deoxy-2-{[(2,2,2-trichloroethoxy)carbonyl]amino}-β-D-glucopyranoside (2) was calculated (HPLC quantitative yield: >99%).

Substrate 1 was subjected to a de-para-methoxyphenylation reaction performed by the same technique as described above using various additives shown in Table 4 below. Note that in the reaction of entry 3, 1.8 equivalents of HTIB were used instead of 1.4 equivalents of HTIB, and 1 volume of water was used instead of 0.5 volumes of water. The post-reaction solution was quantified by HPLC and then the yield of product 2 was calculated (entries 2 and 3).

**[Table 5]**

| Table 4 Results of de-para-methoxyphenylation reaction performed using various additives | | | |
|---|---|---|---|
| Entry | Additive | Time | Yield |
| 1 | NaH₂PO₄ | 2h | >99% |
| 2 | KH₂PO₄ | 24h | 92% |
| 3 | Na₂HPO₄ | 120h | 89% |

### <Example 5>

### Production and isolation of 3,6-di-O-benzyl-2-deoxy-2- { [(2,2,2-trichloroethoxy)carbonyl]amino}-D-glucopyranoside (7b) by de-para-methoxyphenylation reaction

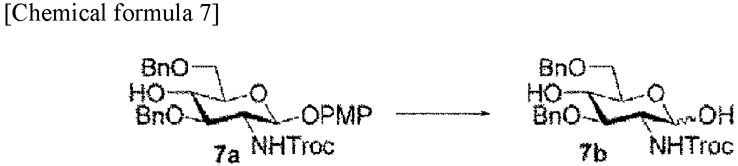

To a solution containing 4-methoxyphenyl 3,6-di-O-benzyl-2-deoxy-2-{[(2,2,2-trichloroethoxy)carbonyl]amino}-β-D-glucopyranoside (7a) (1.00 g, 1.56 mmol), toluene (8 mL, 8 vol.), hexafluoro 2-propanol (HFIP) (5 mL, 5 vol.) and water (0.5 mL, 0.5 vol.), [hydroxy(tosyloxy)iodo]benzene (HTIB) (0.86 g, 2.18 mmol, 1.4 equiv.) was added as λ3-iodane at room temperature (25°C or lower), and then the solution was stirred at the same temperature for 2 hours, thereby performing a de-para-methoxyphenylation reaction. Subsequently, water (8 mL) in which ethyl acetate (25 mL), sodium hydrogen carbonate (0.5 g) and sodium sulfite (0.5 g) had been dissolved was poured, followed by liquid separation, thereby obtaining an organic layer. The obtained organic layer was washed again with water (8 mL) in which sodium hydrogen carbonate (0.5 g) and sodium sulfite (0.5 g) had been dissolved, and further washed with 20% saline (4 mL). The obtained organic layer was dried over sodium sulfate, filtered, and concentrated under reduced pressure. The concentrated residue was purified by silica gel column chromatography (chloroform/methanol = 100/0 to 97/3), and the selected fraction was concentrated under reduced pressure to isolate 3,6-di-O-benzyl-2-deoxy-2-{[(2,2,2-trichloroethoxy)carbonyl]amino}-D-glucopyranoside (7b) (720 mg, 87% isolated yield) as a white solid.

Using 7a as a substrate, [bis(trifluoroacetoxy)iodo]benzene (PIFA) (1.4 equiv.) as λ3-iodane, and dichloromethane as a reaction solvent, a de-para-methoxyphenylation reaction was performed in the same manner as described above. Subsequently, as a result of performing the same treatment as described above, the isolated yield of 7b was 84%.

¹H-NMR (400 MHz, CDCl₃) δ 7.37-7.27 (m, 10H), 5.22 (brs, 1H), 5.17 (d, J = 10.0 Hz, 1H), 4.80 (d, J = 11.6 Hz, 1H), 4.79 (d, J = 12.0 Hz, 1H), 4.72 (d, J = 11.6 Hz, 1H), 4.64 (d, J = 12.0 Hz, 1H), 4.59 (d, J = 12.4 Hz, 1H), 4.54 (d, J = 12.4 Hz, 1H), 4.01 (ddd, J = 7.6, 5.2, 4.0 Hz, 1H), 3.92 (ddd, J = 10.4, 10.4, 4.0 Hz, 1H), 3.80-3.58 (m, 4H), 3.29 (brs, 1H), 2.52 (brs, 1H) .

¹³C-NMR (100 MHz, CDCl₃) δ 154.3, 138.1, 137.6, 128.7, 128.5, 128.2, 128.0,128.0, 116.2, 95.4, 92.2, 79.7, 74.7, 74.6, 73.7, 71.9, 70.2, 54.7, 29.7.

HRMS(ESI-)[M-H]⁻ [C₂₃H₂₅Cl₃NO₇]⁻

Calculated value: 532.0702; Measured value: 532.0701.

### <Example 6>

### Production and isolation of 4-O-acetyl-3,6-di-O-benzyl-2-deoxy-2-(1,3-dioxo-1,3-dihydro-2H-isoindol-2-yl)-D-glucopyranoside (8b) by de-para-methoxyphenylation reaction

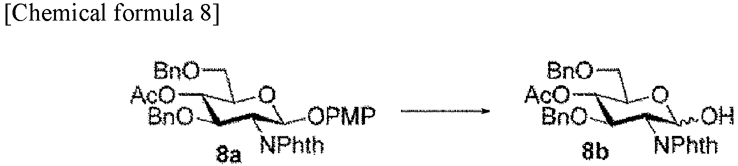

As a result of performing a de-para-methoxyphenylation reaction in the same manner as in Example 5 using 4-methoxyphenyl 4-O-acetyl-3,6-di-O-benzyl-2-deoxy-2-(1,3-dioxo-1,3-dihydro-2H-isoindol-2-yl)-D-glucopyranoside (8a) as a substrate, [hydroxy(tosyloxy)iodo]benzene (HTIB) (1.4 equiv.) as λ3-iodane, and toluene as a reaction solvent, the isolated yield of 8b was 81%.

As a result of performing a de-para-methoxyphenylation reaction in the same manner as in Example 5 using 8a as a substrate, [bis(trifluoroacetoxy)iodo]benzene (PIFA) (1.4 equiv.) as λ3-iodane, and dichloromethane as a reaction solvent, the isolated yield of 8b was 94%.

¹H-NMR (400 MHz, CDCl₃) δ 7.71-7.65 (m, 4H), 7.34-7.26 (m, 5H), 7.01-6.87 (m, 5H), 5.36 (dd, J = 8.0, 8.0 Hz, 1H), 5.13 (dd, J = 8.4, 10.0 Hz, 1H), 4.59 (d, J = 12.4 Hz, 1H), 4.54 (s, 2H), 4.50 (dd, J = 8.4, 10.4 Hz, 1H), 4.33 (d, J = 12.4 Hz, 1H), 4.17 (dd, J = 8.4, 10.4 Hz,1H), 3.79 (ddd, J = 8.4, 5.2, 4.8 Hz, 1H), 3.61-3.53 (m, 2H),3.41 (d, J = 8.0 Hz, 1H), 1.93 (s, 3H) .

¹³C-NMR (100 MHz, CDCl₃) δ 169.8, 168.1, 137.7, 137.7, 134.0, 131.6, 128.4, 128.2,128.0, 127.8. 127.7, 127.5, 123.4, 116.2, 92.9, 73.9, 73.7, 73.5, 72.2, 69.3, 57.1, 20.9.

HRMS(ESI⁻)[M-H]⁻ [C₃₀H₂₈NO₈]⁻

Calculated value:530.1820; Measured value: 530.1841.

### <Example 7>

### Production and isolation of 2,4,6-tri-O-acetyl-3-O-benzyl-D-glucopyranoside (9b) by de-para-methoxyphenylation reaction

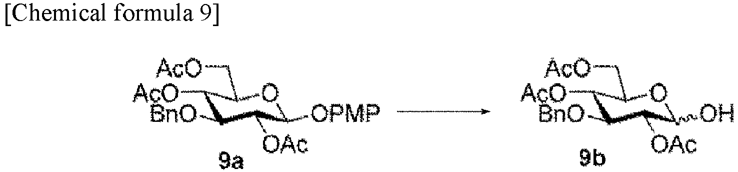

As a result of performing a de-para-methoxyphenylation reaction in the same manner as in Example 5 using 4-methoxyphenyl 2,4,6-tri-O-acetyl-3-O-benzyl-D-glucopyranoside (9a) as a substrate, [hydroxy(tosyloxy)iodo]benzene (HTIB) (1.4 equiv.) as λ3-iodane and toluene as a reaction solvent, the isolated yield of 9b was 97%.

As a result of performing a de-para-methoxyphenylation reaction in the same manner as in Example 5 using 9a as a substrate, [bis(trifluoroacetoxy)iodo]benzene (PIFA) (2.4 equiv.) as λ3-iodane, and dichloromethane as a reaction solvent, the isolated yield of 9b was 98%.

¹H-NMR (400 MHz, CDCl₃) δ 7.37-7.23 (m, 5H), 5.47 (dd, J = 3.6, 3.6 Hz, 1H), 5.10 (dd, J = 9.6, 9.6 Hz, 1H), 4.90-4.84 (m, 1H), 4.71 (d, J = 12.0 Hz,1H), 4.65-4.60 (m, 1H), 4.24-4.01 (m, 3H), 3.75-3.59 (m, 1H), 2.93 (brd, J = 3.6 Hz, 1H), 2.09 (s, 3H), 2.07 (s, 3H), 1.95 (s, 3H) .

¹³C-NMR (100 MHz, CDCl₃) δ 171.1, 170.3, 169.6, 138.2, 128.5, 127.8, 127.6, 90.3, 76.9, 75.0, 73.5, 69.8, 67.7, 62.3, 20.9, 20.8, 20.8.

HRMS(ESI⁻)[M-H]⁻ [C₁₉H₂₃O₉]⁻

Calculated value: 395.1349; Measured value: 395.1344.

### <Example 8>

### Production and isolation of 2,3,4,6-tetra-O-acetyl-D-mannopyranoside (10b) by de-para-methoxyphenylation reaction

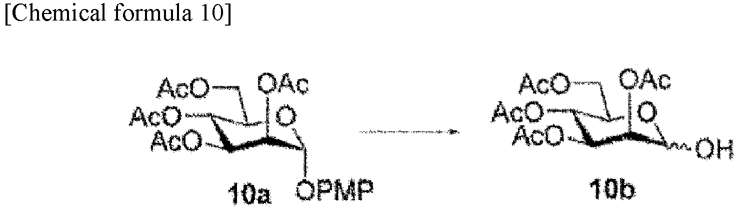

As a result of performing a de-para-methoxyphenylation reaction in the same manner as in Example 5 using 4-methoxyphenyl 2,3,4,6-tetra-O-acetyl-D-mannopyranoside (10a) as a substrate, [bis(trifluoroacetoxy)iodo]benzene (PIFA) (3.5 equiv.) as λ3-iodane, dichloromethane as a reaction solvent, and potassium dihydrogen phosphate (3.0 equiv.) as an additive, the isolated yield of 10b was 88%.

¹H-NMR (400 MHz, CDCl₃) δ 5.41 (dd, J = 3.6, 8.0 Hz, 1H), 5.33-5.01 (m, 3H), 4.43 (s, 1H), 4.29-4.22 (m, 2H), 4.17-4.11 (m, 1H), 2.16 (s, 3H), 2.11 (s, 3H), 2.06 (s, 3H), 2.00 (s, 3H) .

¹³C-NMR (100 MHz, CDCl₃) δ 171.0, 170.4, 170.2, 169.9, 92.0, 70.1, 68.9, 68.3, 66.1, 62.6, 20.9, 20.7, 20.7, 20.7.

HRMS(ESI⁻)[M-H]⁻ [C₁₄H₁₉O₁₀]⁻

Calculated value: 347.0984; Measured value: 347.0999.

### <Example 9>

### Production and isolation of 2-azido-3,6-di-O-benzyl-2-deoxy-D-glucopyranoside (11b) by de-para-methoxyphenylation reaction

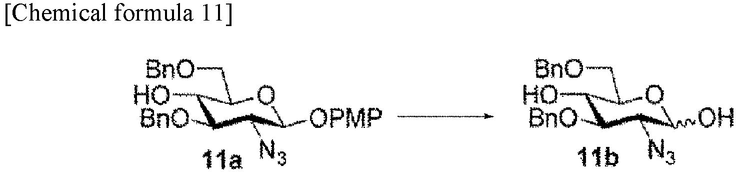

As a result of performing a de-para-methoxyphenylation reaction in the same manner as in Example 5 using 4-methoxyphenyl 2-azido-3,6-di-O-benzyl-2-deoxy-D-glucopyranoside (1 1a) as a substrate, [hydroxy(tosyloxy)iodo]benzene (HTIB) (1.1 equivalent) as λ3-iodane, dichloromethane as a reaction solvent, and potassium dihydrogen phosphate (3.0 equiv.) as an additive, the isolated yield of 11b was 88%.

As a result of performing a de-para-methoxyphenylation reaction in the same manner as in Example 5 using 11a as a substrate, [bis(trifluoroacetoxy)iodo]benzene (PIFA) (1.4 equiv.) as λ3-iodane, and dichloromethane as a reaction solvent, the isolated yield of 1 1b was 79%.

¹H-NMR (400 MHz, CDCl₃) δ 7.40 (m, 10H), 5.20 (d, J = 2.8 Hz, 0.5H), 5.05 (brs, 0.5H), 4.88 (dd, J = 10.8, 2.8 Hz, 1H), 4.76 (d, J = 10.8 Hz, 0.5H), 4.70 (d, J = 10.8 Hz, 0.5H), 4.54 (d, J = 12.0 Hz, 1H), 4.49 (d, J = 12.0 Hz, 1H), 4.43 (brs, 0.5H), 4.37 (d, J = 8.0 Hz, 0.5H), 4.00 (ddd, J = 8.0, 5.2, 3.2 Hz, 0.5H), 3.81 (dd, J = 10.4, 8.8 Hz, 0.5H), 3.67 (ddd, J = 8.0, 6.0, 3.2 Hz, 1H), 3.58 (ddd, J = 6.0, 6.0, 1.2 Hz, 1H), 3.54-3.45 (m, 1H), 3.35 (ddd, J = 6.8, 6.0, 3.2 Hz, 0.5H), 3.27 (m, 1H), 3.15 (m, 0.5H), 2.74 (brd, J = 7.6 Hz, 1H) .

¹³C-NMR (100 MHz, CDCl₃) δ 137.8, 137.8, 137.3, 137.2, 128.5, 128.4, 128.1, 128.0, 128.0, 127.8, 127.8, 95.9, 91.8, 82.4, 79.7, 77.2, 75.0, 74.9, 74.0, 73.5, 73.4, 71.6, 70.9, 69.9, 69.6, 66.5, 63.3.

HRMS(ESI⁻) [M+HCOO]⁻ [C₂₁H₂₄N₃O₇]⁻

Calculated value: 430.1620; Measured value: 430.1638.

### <Example 10>

### Production and isolation of 6-O-{5-acetamido-4,7,8,9-tetra-O-acetyl-3,5-dideoxy-1-methyl-D-glycero-α-D-galacto-non-2-uropyranosyl}-2,4-di-O-benzoyl-3-O-benzyl-D-galactopyranoside (12b)

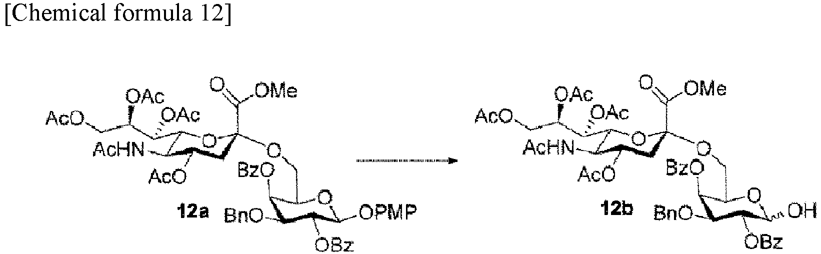

As a result of performing a de-para-methoxyphenylation reaction in the same manner as in Example 5 using 4-methoxyphenyl 6-O-{5-acetamido-4,7,8,9-tetra-O-acetyl-3,5-dideoxy-1-methyl-D-glycero-α-D-galacto-non-2-uropyranosyl}-2,4-di-O-benzoyl-3-O-benzyl-D-galactopyranoside (12a) as a substrate, [bis(trifluoroacetoxy)iodo]benzene (PIFA) (3.5 equiv.) as λ3-iodane, dichloromethane as a reaction solvent, and potassium dihydrogen phosphate (3.0 equiv.) as an additive, the isolated yield of 12b was 94%.

¹H-NMR (400 MHz, CDCl₃) δ 8.14-8.09 (m, 2H), 8.03-7.96 (m, 2H), 7.63-7.12 (m, 11H), 6.70 (m, 1H), 5.99 (brs, 1H), 5.66 (m, 0.5H), 5.55-5.20 (m, 3.5H), 4.84-4.75 (m, 2H), 4.63-4.50 (m, 2H), 4.34 (brd, J = 12.8 Hz, 1H), 4.29 (ddd, J = 10.0, 7.6, 2.0 Hz, 1H), 4.19-3.86 (m, 4.5H), 3.78 (dd, J = 9.6, 4.8 Hz, 0.5H), 3.50-3.18 (m, 4H), 2.55 (m, 1H), 2.17-1.82 (m, 16H) .

¹³C-NMR (100 MHz, CDCl₃) δ 171.7, 171.1, 170.5, 170.3, 170.2, 170.0, 168.0, 167.7, 167.4, 166.1, 165.5, 165.4, 165.2, 149.7, 137.8, 137.3, 137.2, 133.5, 133.3, 133.2, 133.1, 129.9, 129.8, 129.7, 129.5, 128.5, 128.4, 128.3, 128.2, 128.2, 128.0, 127.9, 127.6, 127.5, 116.1, 98.7, 98.7, 98.4, 96.0, 90.9, 77.2, 76.2, 76.0, 72.9, 72.8, 72.7, 72.5, 71.8, 71.5, 71.3, 71.1, 69.9, 69.3, 68.9, 67.7, 67.4, 67.3, 67.3, 67.1, 66.2, 63.3, 62.8, 62.2, 61.8, 52.6, 52.4, 49.1, 37.9, 29.6, 23.0, 21.0, 20.9, 20.8, 20.7, 20.7.

HRMS(ESI⁻) [M+HCOO]⁻ [C₄₈H₅₄NO₂₂]⁻

Calculated value: 996.3143; Measured value: 996.3143.

### <Example 11>

### Production and isolation of 3-O-{5-acetamido-4,7,8,9-tetra-O-acetyl-3,5-dideoxy-1-methyl-D-glycero-α-D-galacto-non-2-uropyranosyl}-2,5,6-tri-O-benzoyl-D-galactopyranoside (13b)

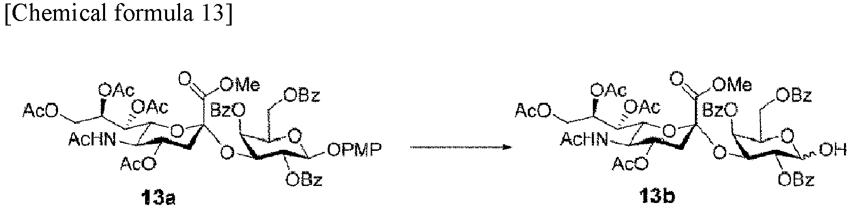

As a result of performing a de-para-methoxyphenylation reaction in the same manner as in Example 5 using 4-methoxyphenyl 3-O-{5-acetamido-4,7,8,9-tetra-O-acetyl-3,5-dideoxy-1-methyl-D-glycero-α-D-galacto-non-2-uropyranosyl}-2,5,6-triO-benzoyl-D-galactopyranoside (13a) as a substrate, [bis(trifluoroacetoxy)iodo]benzene (PIFA) (2.4 equiv.) as λ3-iodane, dichloromethane as a reaction solvent, and potassium dihydrogen phosphate (3.0 equiv.) as an additive, the isolated yield of 13b was 90%.

¹H-NMR (400 MHz, CDCl₃) δ 8.28-8.19 (m, 2H), 8.09-7.93 (m, 4H), 7.60-7.34 (m, 9H), 5.70-5.54 (m, 2H), 5.50 (d, J = 2.4 Hz, 0.5H), 5.44 (d, J = 2.4 Hz, 0.5H), 5.40-5.20 (m, 2.5H), 5.15-5.05 (m, 1H), 4.99 (dd, J = 10.0, 3.2 Hz, 0.5H), 4.84 (m, 1H), 4.63-4.55 (m, 1H), 4.47 (ddd, J = 11.6, 11.2, 6.0 Hz, 1H), 4.40-4.24 (m, 3H), 4.09-3.91 (m, 2H), 3.82 (s, 3H), 3.68 (ddd, J = 10.8, 4.8, 2.4 Hz, 1H), 2.46 (dd, J = 12.8, 4.0 Hz, 1H), 2.27-1.62 (m, 15H) .

¹³C-NMR (100 MHz, CDCl₃) δ 171.5, 171.0, 170.9, 170.8, 170.6, 170.4, 170.3, 170.1, 170.1, 169.7, 169.6, 168.1, 168.1, 167.0, 165.9, 165.9, 165.7, 165.6, 165.4, 165.2, 133.5, 133.5, 133.3, 133.3, 133.3, 133.1, 133.1, 130.2, 130.1, 129.9, 129.8, 129.8, 129.7, 129.6, 129.5, 129.4, 129.3, 129.2, 128.5, 128.5, 128.2, 118.9, 114.3, 97.2, 97.0, 96.9, 96.8, 96.0, 92.1, 91.9, 77.2, 73.3, 72.5, 72.4, 72.1, 71.0, 70.9, 69.8, 69.4, 69.3, 68.8, 68.6, 68.4, 68.0, 67.6, 67.5, 67.2, 67.0, 66.8, 66.7, 66.5, 62.6, 62.5, 62.4, 62.3, 61.5, 53.2, 53.1, 49.8, 48.9, 38.1, 37.4, 37.4, 23.1, 23.1, 21.4, 21.4, 21.1, 20.8, 20.7, 20.6, 20.6, 20.5.

HRMS(ESI⁺) [M+H]⁺ [C₄₇H₅₂NO₂₁]⁺

Calculated value: 966.3026; Measured value: 966.3024.

### <Example 12>

### Production and isolation of 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-D-glucopyranosyl}-β-D-glucopyranoside (14b)

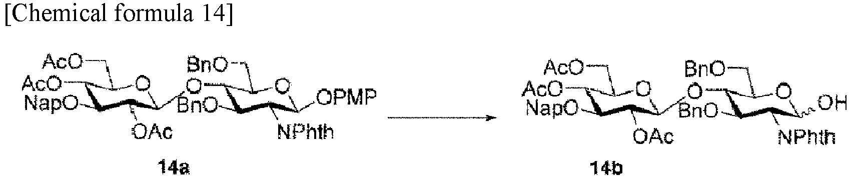

As a result of performing a de-para-methoxyphenylation reaction in the same manner as in Example 5 using 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-D-glucopyranosyl}-β-D-glucopyranoside (14a) as a substrate, [bis(trifluoroacetoxy)iodo]benzene (PIFA) (1.5 equiv.) as λ3-iodane, dichloromethane as a reaction solvent, and potassium dihydrogen phosphate (3.0 equiv.) as an additive, the isolated yield of 14b was 95%.

¹H-NMR (400 MHz, CDCl₃) δ 7.82 (m, 3H), 7.68-7.62 (m, 5H), 7.48 (m, 2H), 7.40-7.27 (m, 8H), 6.99 (m, 2H), 6.80 (m, 3H), 5.30 (d, J = 8.8 Hz, 1H), 5.11 (t, J = 9.6 Hz, 1H), 5.06 (dd, J = 9.6, 8.4 Hz, 1H), 4.78 (dd, J = 13.2, 7.2 Hz, 2H), 4.74 (d, J = 11.6 Hz, 1H), 4.70 (d, J = 11.6 Hz, 1H), 4.52 (d, J = 8.4 Hz, 1H), 4.42 (dd, J = 12.0, 15.6 Hz, 2H), 4.26 (dd, J = 10.8, 8.4 Hz, 1H), 4.18 (dd, J = 12.4, 4.8 Hz, 1H), 4.03 (dd, J = 10.8, 8.8 Hz, 2H), 3.96 (dd, J = 12.4, 2.4 Hz, 1H), 3.78 (dd, J = 11.2, 3.6 Hz, 1H), 3.72 (brd, J = 11.2 Hz, 1H), 3.54 (t, J = 9.6 Hz, 1H), 3.39 (ddd, J = 7.6, 4.4, 2.0 Hz, 1H), 1.96 (s, 3H), 1.93 (s, 3H), 1.91 (s, 3H) .

¹³C-NMR (100 MHz, CDCl₃) δ 170.8, 169.3, 168.9, 167.9, 138.5, 137.8, 135.2, 133.7, 133.1, 132.9, 131.5, 128.5, 128.2, 128.1, 127.9, 127.9, 127.8, 127.6, 127.0, 126.2, 126.2, 126.0, 125.5, 123.2, 100.3, 92.8, 80.3, 78.1, 76.3, 74.7, 74.5, 73.8, 73.6, 72.8, 71.7, 69.6, 67.6, 61.9, 57.4, 20.8, 20.7, 20.6.

HRMS(ESI⁻) [M-H]⁻ [C₅₁H₅₀NO₁₅]⁻

Calculated value: 916.3186; Measured value: 916.3204.

### <Example 13>

### Production and isolation of 3,6-di-O-benzyl-2-deoxy-4-O-{6-O-acetyl-2,4-di-O-benzyl-3-O-[(naphthalen-2-yl)methyl]-β-D-mannopyranosyl}-2-(1,3-dioxo-1,3-dihydro-2H-isoindol-2-yl)-D-glucopyranoside (15b)

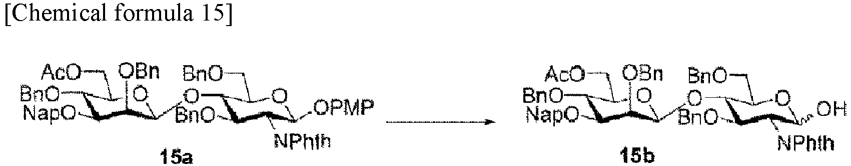

As a result of performing a de-para-methoxyphenylation reaction in the same manner as in Example 5 using 4-methoxyphenyl 3,6-di-O-benzyl-2-deoxy-4-O-{6-O-acetyl-2,4-di-O-benzyl-3-O-[(naphthalen-2-yl)methyl]-β-D-mannopyranosyl}-2-(1,3-dioxo-1,3-dihydro-2H-isoindol-2-yl)-D-glucopyranoside (15a) as a substrate, [bis(trifluoroacetoxy)iodo]benzene (PIFA) (2.5 equiv.) as λ3-iodane, dichloromethane as a reaction solvent, and potassium dihydrogen phosphate (3.0 equiv.) as an additive, the isolated yield of 15b was 81%.

¹H-NMR (400 MHz, CDCl₃) δ 7.84-7.37 (m, 13H), 7.36-7.17 (m, 13H), 6.88-6.85 (m, 2H), 6.77-6.71 (m, 3H), 5.32 (brd, J = 7.6 Hz, 1H), 4.93-4.85 (m, 4H), 4.64 (d, J = 12.4 Hz, 1H), 4.60 (d, J = 12.4 Hz, 1H), 4.56 (d, J = 7.2 Hz, 1H), 4.53 (d, J = 8.4 Hz, 1H), 4.50 (s, 1H), 4.44 (d, J = 12.8 Hz, 1H), 4.35-4.21 (m, 4H), 4.05 (dd, J = 10.8, 8.4 Hz, 1H), 3.99 (dd, J = 8.8, 8.8 Hz, 1H), 3.83 (dd, J = 10.0, 10.0 Hz, 1H), 3.77 (d, J = 2.8 Hz, 1H), 3.65-3.51 (m, 4H), 3.41-3.35 (m, 2H), 1.88 (s, 3H) .

¹³C-NMR (100 MHz, CDCl₃) δ 170.9, 168.0, 138.8, 138.7, 138.0, 137.6, 135.5, 133.6, 133.2, 132.9, 131.5, 128.5, 128.4, 128.1, 128.0, 127.9, 127.8, 127.7, 127.7, 127.7, 127.4, 127.3, 126.8, 126.2, 125.9, 125.5, 123.2, 101.5, 92.9, 82.4, 79.2, 77.2, 76.9, 75.0, 74.7, 74.6, 74.4, 74.0, 73.5, 73.4, 71.7, 68.5, 63.4, 57.5, 20.7.

HRMS(ESI⁻) [M+HCOO]⁻ [C₆₂H₆₀NO₁₅]⁻

Calculated value: 1058.3968; Measured value: 1058.3933.

Table 5 below shows the conditions and results of the de-para-methoxyphenylation reaction based on the λ3-iodane-HFIP system using monosaccharides as substrates in Examples 5 to 9 above. Table 6 below shows the conditions and results of the de-para-methoxyphenylation reaction based on the λ3-iodane-HFIP system using disaccharides as substrates in Examples 10 to 13 above. All deprotected products were obtained with good yields. Moreover, as shown in Table 6, the deprotected products were also obtained with high yields with the use of disaccharides, similarly to the results of the use of monosaccharides.

**[Table 6]**

| Table 5 Conditions and results of de-para-methoxyphenylation reaction based on λ3-iodane-HFIP system using monosaccharides as substrates (Examples 5 to 9) | | | | |
|---|---|---|---|---|
| 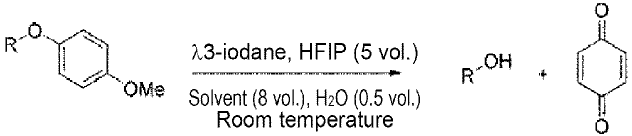 | | | | |
| Entry | λ3-iodane /solvent | Substrate | Product | Yield |
| 1 | HTIB (1.4 equiv.) /toluene | 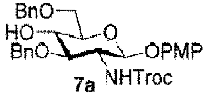 | 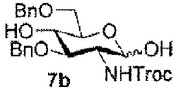 | 87% |
| 2 | PIFA (1.4 equiv.) /DCM | 7a | 7b | 84% |
| 3 | HTIB (1.4 equiv.) /toluene | 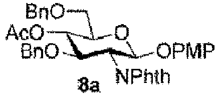 | 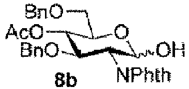 | 81% |
| 4 | PIFA 1.4 equiv.) /DCM | 8a | 8b | 94% |
| 5 | HTIB (1.4 equiv.)/ toluene | 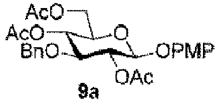 | 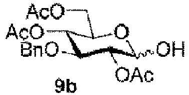 | 97% |
| 6 | PIFA (2.4 equiv.) /DCM | 9a | 9b | 98% |
| 7 | PIFA (3.5 equiv.)/DCM Additive: KH₂PO₄ (3 equiv.) | 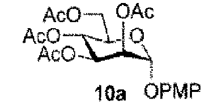 | 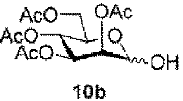 | 88% |
| 8 | HTIB (1.1 equiv.)/DCM, Additive: KH₂PO₄ (3 equiv.) | 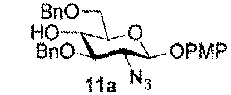 | 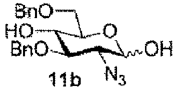 | 88% |
| 9 | PIFA (1.4 equiv.) /DCM | 11a | 11b | 79% |

**[Table 7]**

| Table 6 Conditions and results of de-para-methoxyphenylation reaction based on λ3-iodane-HFIP system using disaccharides as substrates (Examples 10 to 13) | | | | |
|---|---|---|---|---|
| 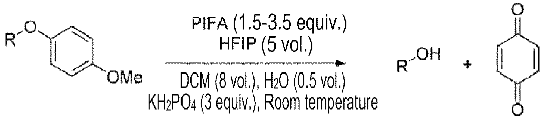 | | | | |
| Entry | **PIFA** | Substrate | Product | Yield |
| 1 | 3.5 equiv. | 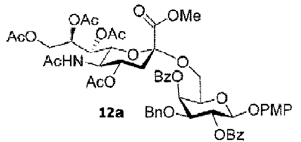 | 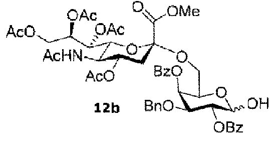 | 94% |
| 2 | 2.4 equiv. | 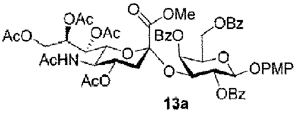 | 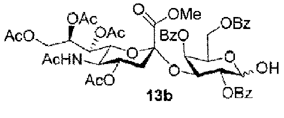 | 90% |
| 3 | 1.5 equiv. | 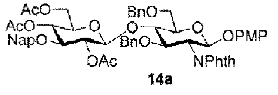 | 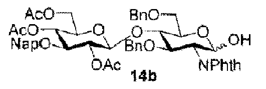 | 95% |
| **4** | 2.5 equiv. | 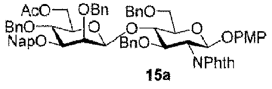 | 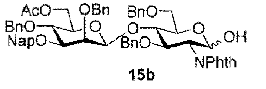 | 81% |

### <Example 14>

### De-para-methoxyphenylation reaction of para-methoxyphenyl-protected benzyl alcohol

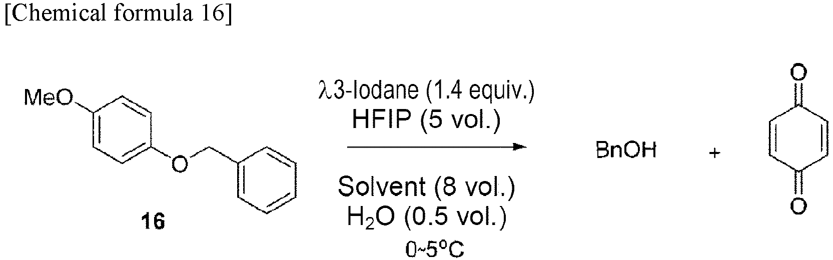

To a solution containing 4-benzyloxyanisole (16) (100 mg, 0.467 mmol), toluene (0.8 mL), hexafluoro-2-propanol (HFIP) (0.5 mL) and water (0.05 mL), [hydroxy(tosyloxy)iodo]benzene (HTIB) (0.26 g, 0.653 mmol) was added as λ3-iodane at 5°C, and then the solution was stirred at the same temperature for 0.5 hours. This solution was quantified by HPLC, and then the yield of the obtained p-benzoquinone was calculated (HPLC quantitative yield: benzyl alcohol (BnOH)>99%, p-benzoquinone 67%).

To a solution containing 4-benzyloxyanisole (16) (100 mg, 0.467 mmol), toluene (0.8 mL), dichloromethane (0.5 mL) and water (0.05 mL), [bis(trifluoroacetoxy)iodo]benzene (PIFA) (1.4 equiv.) was added as λ3-iodane at 5°C, and then the solution was stirred at the same temperature for 0.5 hours. This solution was quantified by HPLC, and the yield of the obtained p-benzoquinone was calculated (HPLC quantitative yield: benzyl alcohol (BnOH) 99%, p-benzoquinone 75%).

Table 7 below summarizes the above de-para-methoxyphenylation reaction conditions and product yields. All deprotected products were obtained with good yields.

**[Table 8]**

| Table 7 De-para-methoxyphenylation reaction of 4-benzyloxyanisole (16) by the use of λ3-iodane-HFIP system | | | | | |
|---|---|---|---|---|---|
| Entry | λ3-iodane | Solvent | Time | Br0H | p-Benzoquinone |
| 1 | HTIB | Toluene | 0.5h | >99% | 61% |
| 2 | PIFA | CH₂Cl₂ | 2h | 99% | 75% |

The measurement of yields by HPLC analysis in Example 14 was performed under the following analytical conditions.

### <HPLC analytical conditions>

Equipment used: SHIMAZU HPLC (2010A HT)
Column: Xbrige C18 3.5 µm, 4.6x150 mm (Waters)
Mobile phase A: 10 mM AcONH₄ aqueous solution
Mobile phase B: CH₃CN

### Gradient conditions:

**[Table 9]**

| Time/solvent | 10 mM AcONH₄ aqueous solution | CH₃CN |
|---|---|---|
| 0 min | 85% | 15% |
| 7. 5 min | 50% | 50% |
| 10.5 min | 50% | 50% |
| 18.0 min | 5% | 95% |
| 23.0 min | 5% | 95% |

Flow rate: 1 mL/min.
Detection wavelength: 210 nm
Column temperature: 40°C
Injection volume: 5 µL
Retention time: 19.4 minutes(16), 7.6 minutes (benzyl alcohol), 5.0 minutes (p-benzoquinone), 14.6 minutes (iodobenzene)

## Claims

1. A method for producing a compound represented by the formula R-OH, comprising a step of reacting a compound represented by the formula R-OX with λ3-iodane in a fluorous alcohol and water, wherein R is a substrate, X is a phenyl group substituted by C₁ to C₅ alkoxy at the para- or ortho-position, and the phenyl group may optionally be further substituted.

2. The method according to claim 1, wherein the C₁ to C₅ alkoxy in the X group is methoxy, ethoxy, propyloxy, or isopropyloxy.

3. The method according to claim 1, wherein the C₁ to C₅ alkoxy in the X group is paramethoxy.

4. The method according to any one of claims 1 to 3, wherein the λ3-iodane is a compound represented by the formula R¹-I(OR²)₂, wherein R¹ is an unsubstituted or substituted phenyl group, and R² is selected from the group consisting of H, acetyl, trifluoroacetyl, tosyl, methanesulfonyl, and a combination thereof.

5. The method according to claim 4, wherein the compound represented by the formula R¹-I(OR²)₂ is selected from the group consisting of [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, [hydroxy(methanesulfonyloxy)iodo]benzene, and a combination thereof.

6. The method according to any one of claims 1 to 5, wherein the amount of the λ3-iodane is 0.1 to 10 equivalents relative to the substrate.

7. The method according to any one of claims 1 to 6, wherein the fluorous alcohol is a fluorous aliphatic alcohol.

8. The method according to claim 7, wherein the fluorous aliphatic alcohol is a fluorous C₂ to C₈ aliphatic alcohol.

9. The method according to claim 8, wherein the fluorous C₂ to C₈ aliphatic alcohol is selected from the group consisting of hexafluoro 2-propanol (HFIP), 2,2,2-trifluoroethanol (TFE), 2,2,3,3,4,4,5,5-octafluoro-1-pentanol, nonafluoro-tert-butyl alcohol, and a combination thereof.

10. The method according to any one of claims 1 to 9, comprising adding a solvent selected from the group consisting of CH₂Cl₂, toluene, (trifluoromethyl)benzene, and a combination thereof.

11. The method according to any one of claims 1 to 10, wherein the amount of the fluorous alcohol is 1.0 equivalent or more in a molar ratio and 15 or less in a volume ratio relative to the substrate.

12. The method according to any one of claims 1 to 11, wherein the amount of the water is 1.0 equivalent or more in a molar ratio and 10 or less in a volume ratio relative to the substrate.

13. The method according to any one of claims 1 to 12, comprising adding an additive selected from the group consisting of sodium dihydrogen phosphate (NaH₂PO₄), potassium dihydrogen phosphate (KH₂PO₄), disodium hydrogen phosphate (Na₂HPO₄), and a combination thereof.

14. The method according to any one of claims 5 to 13, comprising adding trifluoroacetic acid when (diacetoxyiodo)benzene (PIDA) is used as the λ3-iodane.

15. The method according to any one of claims 1 to 14, which is performed at - 20°C to 60°C.

16. The method according to any one of claims 1 to 15, wherein the substrate R is a sugar having the OX group at the 1-position or the anomeric position.

17. The method according to claim 16, wherein the sugar is a monosaccharide or a polysaccharide.

18. The method according to claim 17, wherein the monosaccharide has a 5-membered ring or 6-membered ring structure.

19. The method according to claim 18, wherein the monosaccharide is a pentose or a hexose.

20. The method according to claim 19, wherein the hexose is glucose, mannose, galactose, or glucosamine.

21. The method according to claim 17, wherein the polysaccharide is a disaccharide to a decasaccharide.

22. The method according to claim 17, wherein the polysaccharide is (i) a disaccharide that is galactose-glucosamine, glucosamine-glucosamine, neuraminic acid-galactose, or mannose-glucosamine, (ii) a trisaccharide consisting of two mannoses and one glucosamine, or a trisaccharide consisting of neuraminic acid, galactose and glucosamine, or (iii) a tetrasaccharide consisting of two mannoses and two glucosamines, or a tetrasaccharide consisting of three mannoses and one glucosamine.

23. The method according to any one of claims 16 to 22, wherein a hydroxy group at a carbon adjacent to the carbon at the 1-position or the anomeric position in the sugar is protected with an acyl group, or an amino group at a carbon adjacent to the carbon at the 1-position or the anomeric position in the sugar is protected with an imide group, an acyl group, or a carbamate group, or a carbon adjacent to the carbon at the 1-position or anomeric position in the sugar has an azide group (N₃).

24. The method according to claim 23, whereinthe imide group as the amino group-protecting group is phthaloyl (Phth), the acyl group is acetyl group (Ac), and the carbamate group is selected from the group consisting of (2,2,2-trichloroethoxy)carbonyl (Troc), allyloxycarbonyl (Alloc), 2-(trimethylsilyl)ethoxycarbonyl (Teoc), 9-fluorenylmethyloxycarbonyl (Fmoc), tert-butoxycarbonyl (Boc), and benzyloxycarbonyl (Cbz).

25. The method according to claim 23, wherein the acyl group as the hydroxy group-protecting group is selected from the group consisting of acetyl (Ac) and benzoyl (Bz).

26. A method for producing a sugar to which one or two or more attaching moieties are bound, comprising a step of binding one or two or more attaching moieties to a sugar obtained by the method according to any one of claims 16 to 25, wherein the one or two or more attaching moieties are selected from the group consisting of a protein, a nucleic acid molecule, a lipid molecule, a eukaryotic cell, a prokaryotic cell, a tissue derived from an organism, a virus, a parasite, a low-molecular-weight compound, and an artifact.

27. The method according to claim 26, wherein the attaching moiety is a protein.

28. The method according to claim 27, wherein the protein is a receptor, and wherein the receptor is a soluble receptor, fused to the Fc region of an antibody, or unmodified.

29. The method according to claim 27, wherein the protein is an antibody or an antigen-binding fragment thereof, and wherein the antibody or the antigen-binding fragment thereof is bound to a peptide, a nucleic acid molecule, a lipid molecule, a low-molecular-weight compound, an artifact, another antibody or an antigen-binding fragment thereof, or a toxin, or forms a complex with a drug, or is unmodified.

30. The method according to claim 27, wherein the protein is a cytokine, and wherein the cytokine is bound to an antibody or an antigen-binding fragment thereof, or is unmodified.

31. The method according to any one of claims 26 to 30, comprising a step of binding to the sugar one or two or more additional attaching moieties other than the attaching moiety that is a protein.

32. The method according to any one of claims 1 to 15, wherein the substrate R is Ar-(CR¹R²)n-, wherein n = 1 to 3, Ar is an aromatic ring, R¹ and R² are each H, an aromatic ring, or an aliphatic group, and wherein any of the aromatic ring and the aliphatic group may be optionally substituted.

33. The method according to claim 32, wherein the aromatic ring is a C₅ to C₂₀ aryl group or a 5- to 20-membered ring heteroaryl group that may be optionally substituted, and wherein the aliphatic group is a C₁ to C₁₀ aliphatic hydrocarbon group that may be optionally substituted.

34. The method according to claim 32 or 33, wherein the aromatic ring is selected from the group consisting of xylene, toluene, styrene, ethylbenzene, cumene, furan, thiophene, pyrrole, pyran, thiopyran, pyridine, thiazole, imidazole, pyrimidine, 1,3,5-triazine, naphthalene, indene, anthracene, phenanthrene, fluorene, biphenyl, triphenyl, terphenyl, binaphthyl, phenyl naphthalene, indole, quinoline, and purine.
